# EUROPEAN PATENT APPLICATION

(11) **EP 4 309 649 A1**
(43) Date of publication of application: **24.01.2024**
(21) Application number: 22382690.0
(22) Date of filing: 19.07.2022
(51) Int. Cl.: A61K 9/48, A61K 47/38, A61K 47/42, A61K 47/02

(54) **A TITANIUM DIOXIDE FREE HARD CAPSULE SHELL**

(71) Applicant: Qualicaps Europe, S.A., 28108 Alcobendas (ES)
(72) Inventor: NAVARRO RUIZ, Eva, 28108 ALCOBENDAS (ES); ECENARRO PROBST, Susana, 28108 ALCOBENDAS (ES)
(74) Representative: ZBM Patents - Zea, Barlocci & Markvardsen

(57) **Abstract**

It is provided a TiO₂ free hard capsule shell comprising a water-soluble film forming compound selected from the group consisting of a water-soluble cellulose compound and gelatin; optionally, a gelling agent; optionally, a gelling aid; ZnO, and CaCO₃, wherein ZnO and CaCO₃ are in a suitable amount to provide a white color to the film. It is also provided a TiO₂ free suspension for preparing the hard capsule; and a capsule product comprising the TiO₂ free hard capsule filled with a formulation comprising an active ingredient.

## Description

### Technical Field

The present invention is related to the field of hard capsules. In particular, the invention relates to a TiO₂ free hard capsule shell comprising ZnO and CaCO₃, to a suspension for preparing the hard shell capsule, a method for preparing a TiO₂ free hard shell capsule, and a capsule product comprising the TiO₂ free hard shell capsule filled with a formulation comprising an active ingredient.

### Background Art

Hard capsule shells are commonly used for the preparation of oral formulations containing active ingredients such as drugs, nutrition supplements, and so on. In order to prevent the degradation of light sensitive active ingredients, hard capsule shells are made opaque to light. Titanium dioxide has typically been used as colorant and opacifier in the preparation of white hard capsule shells. Nevertheless, recent regulations will restrict the use of titanium dioxide as a food additive, and particularly in capsule shells, in the European Union.

Thus, there is a need for finding an alternative to titanium dioxide as a white colorant and opacifier in hard capsule shells which allows achieving a good balance between opacity and mechanical strength of the capsule shell.

### Summary of Invention

The inventors have found that the use of a composition comprising ZnO and CaCO₃ instead of TiO₂ as white colorant on a hard capsule film allows providing a hard capsule shell having a good light-shielding capacity, while maintaining the mechanical strength required for producing, processing, and filling with active ingredient. Thus, the hard capsule shells of the present invention are TiO₂ free capsule shells and are defined herein below. Surprisingly, the hard capsule shells of the invention have some improved mechanical properties, such as a higher elongation at break, compared to hard capsule shells made of a film comprising TiO₂, while having a high lightness and the required opacity. Particularly, the hard capsule shells of the invention are less fragile and more elastic the TiO₂ hard capsule shells.

Additionally, the hard capsule shells of the invention have a slight basic pH, which has the additional advantage of contributing to inhibiting microbial growth.

Therefore, a first aspect of the present disclosure relates to a TiO₂ free hard capsule shell comprising
(1) a water-soluble film forming compound selected from the group consisting of a water-soluble cellulose compound and gelatin,
(2) optionally, a gelling agent,
(3) optionally, a gelling aid,
(4) ZnO, and
(5) CaCO₃,
wherein ZnO and CaCO₃ are in a suitable amount to provide a white color to the film.

A further advantage is that the TiO₂ free hard capsule shells of the invention provide calcium and zinc that are essential nutrients being among the most important minerals in human nutrition.

A second aspect of the present disclosure relates to a TiO₂ free hard capsule shell-preparing suspension comprising
(1) a water-soluble film forming compound selected from the group consisting of a water-soluble cellulose compound and gelatin,
(2) optionally, a gelling agent,
(3) optionally, a gelling aid,
(4) ZnO,
(5) CaCO₃, and
(6) an aqueous solvent,
wherein the suspension is for the preparation of a hard capsule shell, and ZnO and CaCO₃ are in a suitable amount to provide a white color to the film.

Advantageously, the suspension comprising ZnO and CaCO₃, used for the preparation of the hard capsule shells of the invention, has a lower viscosity than a similar suspension comprising TiO₂ instead of the mixture comprising ZnO and CaCO₃. This lower viscosity is an advantage for the manufacturing process, since it eases the passage of the suspension through the different tubes and tanks connected to the capsule producing machine. Besides, the suspension has a lower gelling temperature which results in lower production process energy costs.

A further aspect of the invention relates to a capsule product comprising the TiO₂ free hard capsule shell as defined herein above and below filled with a formulation comprising an active ingredient.

### Brief Description of Drawings

Fig. 1 shows a Secondary Electron Images (SEI) obtained by Scanning Electron Microscopy (SEM) of zinc oxide powder at 25,000x by applying an accelerating voltage of 20 kV, at a working distance (WD) of 15.0 mm.
Fig. 2 shows a SEI image obtained by SEM of calcium carbonate powder at 25,000x; 20.0 kV, WD 15.0 mm.
Fig. 3 shows a SEI image obtained by SEM of rice starch powder at 1,500x; 20.0 kV, WD 15.0 mm.
Fig. 4 shows a SEI image obtained by SEM of TiO₂ at 15,000x; 20.0 kV, WD 15.0 mm. Fig. 5 shows a SEI image obtained by SEM of a film prepared with the composition of Comparative example 1 (i.e. without colorant) at 1,500x, wherein the presence of KCI particles can be observed; 20.0 kV, WD 15.0 mm.
Fig. 6 shows a SEI image obtained by SEM of a film prepared with the composition of Comparative Example 2 (i.e. a composition comprising TiO₂ as colorant) at 500x; 5.0 kV, WD 15.0 mm.
Fig. 7 shows a SEI image obtained by SEM of a film prepared with the composition of Example 1 (i.e. a TiO₂ free composition containing as colorant a 5 wt.% of a composition of 46 wt.% ZnO, 40 wt.% CaCO₃, and 14 wt.% rice starch) at 500x; 5.0 kV, WD 15.0 mm.
Fig. 8 shows a SEI image obtained by SEM of a film prepared with the composition of Comparative Example 2 at 1,500x; 5.0 kV, WD 15.0 mm).
Fig. 9 shows a SEI image obtained by SEM of a film prepared with the composition of Example 1 at 1500x; 5.0 kV, WD 15.0 mm.
Fig. 10 shows a SEI image obtained by SEM of a film prepared with the composition of Comparative Example 2 at 10,000x; 5.0 kV, WD 15.0 mm).
Fig. 11 shows a SEI image obtained by SEM of a film prepared with the composition of Example 1 at 10,000x; 5.0 kV, WD 15.0 mm.
Fig. 12 shows an image obtained with a fluorescence Zeiss microscope at 10x of a film prepared with the composition of Comparative example 1 (i.e. without colorant).
Fig. 13 shows an image obtained with a fluorescence Zeiss microscope at 10x of the distribution of TiO₂ in the structure of the film prepared with the composition of Comparative Example 2 (containing TiO₂ as colorant) observed at 10x.
Fig. 14 an image obtained with a fluorescence Zeiss microscope at 10x of the distribution of the colorant in the structure of the film prepared with the composition of Example 1 (containing a colorant comprising ZnO and CaCO₃) observed at 10x.

### Detailed description of the invention

All the terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition. In addition, for the purposes of the present invention, any ranges given include both the lower and the upper end-points of the range. Ranges given should be considered approximate, unless specifically stated.

The term "hard capsule shell", as used herein, refers to a two-piece capsule composed of a cap portion and a body portion and specifically refers to an empty capsule, which is intended to be filled with a content.

The term "film forming compound" as used herein can refer to a material used as base for hard capsule shells. Examples include a water-soluble cellulose compound such as HPMC and gelatin.

The term "average", as used herein, refers to the arithmetic mean, that is the sum of the values divided by the number of values being averaged.

The terms "particle size" and "particle size distribution", as used herein, are in terms of diameter irrespective of the actual particle shape. The term "diameter", as used herein, means the equivalent sphere diameter, namely the diameter of a sphere having the same diffraction pattern, when measured by laser diffraction, as the particle.

The term "D50 average particle size", or "Dv50", or volume basis median particle size indicates that in the particle mixture, the diameter of 50% (on the total mass) of the particles is smaller than the indicated D50 average size. Typical measurement techniques are sieve analysis, direct imaging, and laser diffraction known in the art. Particularly, particle size can be determined using laser diffraction method (Mastersizer 2000, available from Malvern Instruments Ltd). Mastersizer^{™} apparatus available from Malvern Instruments Ltd. The measurements can be done on dry powder dispersed using an air pressure of 2 bar.

As used herein, the indefinite articles "a" and "an" are synonymous with "at least one" or "one or more." Unless indicated otherwise, definite articles used herein, such as "the," also include the plural of the noun.

The term "room temperature" refers to a temperature of about 20 °C to about 25 °C.

As mentioned above, the hard capsule shell of the present invention comprises a water-soluble film forming compound, optionally, a gelling agent, optionally, a gelling aid, and a mixture of ZnO, and CaCO₃ in a suitable amount to provide a white color to the film.

CaCO₃ can be ground calcium carbonate (GCC), encapsulated calcium carbonate (ECC), or precipitated calcium carbonate (PCC).

In one embodiment, the CaCO₃ is PCC. Particularly, PCC has a substantially round or prismatic particle shape. In a particular embodiment, the calcium carbonate encompasses small round or prismatic uniform particles.

In a particular embodiment, optionally in combination with one or more features of the embodiments defined above, the PCC has an average particle size (Dv50) from 0.2 to 4.0 µm. In an embodiment, the PCC has a median particle size from from 0.5 to 3.5 µm. In another embodiment, the PCC has a median particle size from 0.8 to 2.5 µm, preferably from 1.0 to 1.8 µm. PCCs from the mentioned particle sizes are commercially available.

PCC can be prepared from calcium oxide (CaO - lime), whereto water is added to give calcium hydroxide. Subsequently carbon dioxide is passed through this solution to precipitate the desired calcium carbonate, known in the industry as PCC. PCC is available in numerous crystal morphologies and sizes. The calcium oxide used as starting material for the precipitation process can be obtained through the milk of lime process, which encompasses crushing of high purity calcium carbonate rock into small particles or powder suitable for processing, heating the latter to about 1000 °C, thereby taking the calcium carbonate apart into calcium oxide (CaO) and carbon dioxide (CO₂) which can be captured and reused in the precipitation process above.

In an embodiment, optionally in combination with one or more features of the embodiments defined above, the water-soluble film forming compound is a water-soluble cellulose compound. Particularly, the film forming compound is a water-soluble cellulose ether in which one or more hydrogen atoms of hydroxyl groups of cellulose are replaced with at least one group selected from alkyl and hydroxyalkyl.

The "alkyl groups" in the alkyl groups or hydroxyalkyl groups refers linear or branched lower alkyl groups having 1 to 6 carbon atoms, and preferably 1 to 4 carbon atoms. Specific examples include methyl, ethyl, butyl, and propyl. Specific examples of water-soluble cellulose compounds include methyl cellulose, hydroxymethyl cellulose, hydroxyethyl cellulose, hydroxypropyl cellulose, hydroxyethyl methylcellulose, hydroxyethyl ethylcellulose, and hydroxypropyl methylcellulose (herein sometimes also referred to as hypromellose or HPMC). The mentioned water-soluble cellulose compounds are commercially available. Among these cellulose compounds, HPMC is particularly preferable because of its excellent film-forming properties and mechanical strength under low-moisture conditions.

Examples of commercially available HPMC include the TC-5 series, the SB-4 (trademark) series, and the METOLOSE (trademark) series of Shin-Etsu Chemical Co., Ltd.; the AnyCoat-C (trademark) series of Lotte (formerly Samsung) Precision Chemistry Co. Ltd.; and the Methocel (trademark) series of The Dow Chemical Company. Further, the hypromellose that can be used in the present invention includes hypromellose having a viscosity of 3 to 15 mPa·s, or of 3 to 10 mPa·s, or of 3 to 8 mPa·s, as measured at 20 °C ± 0.1 °C in the form of a 2 wt% aqueous solution of hypromellose.

A skilled person in the art will know which water-soluble cellulose compound such as which specific hypromellose to use depending on the specific intended use of the hard capsule shell.

In another embodiment, optionally in combination with one or more features of the embodiments defined above, the water-soluble film-forming compound is gelatin. When gelatin is used as film-forming compound, there is no need to use gelling agent (water is considered to be the gelling agent), and, thus, no need to use gelling aid either.

The amount of water-soluble film forming compound contained in the hard capsule shell of the present invention is not limited, as long as the hard capsule shell can be formed by the cold gelation method (disclosed herein below). The amount of water-soluble film forming compound can be, for example, from 75.5 to 97.7 wt.%, preferably from 81.7 to 95.3 wt.%, more preferably from 84.7 to 94.1 wt.%, based on 100 wt.% of water-soluble film forming compound, optionally, gelling agent, optionally, the gelling aid, ZnO, and CaCO₃.

In an embodiment, optionally in combination with one or more features of the embodiments defined above, the gelling agent is selected from the group consisting of carrageenan, pectin, xanthan gum, locust bean gum, tamarind seed polysaccharide, curdlan, fur selenium, agar, gellan gum, and combinations thereof. Preferably, the gelling agent is carrageenan, more particularly kappa-carrageenan.

Among the above gelling agents, carrageenan has a high gel strength. Furthermore, carrageenan, even when used in a small amount, can provide an excellent gelation effect in the presence of specific ions. Therefore, carrageenan is the most preferable gelling agent. In general, three types of carrageenan are known: kappa-carrageenan, iota-carrageenan, and lambda-carrageenan. In the present invention, kappa-carrageenan and iota-carrageenan with relatively high hardness and gelation ability can be preferably used. Pectin can be classified into low methoxyl (LM) pectin and high methoxyl (HM) pectin, according to the difference in the degree of esterification. Gellan gum can also be classified into acylated gellan gum (native gellan gum) and deacylated gellan gum, according to the presence or absence of acylation. In the present invention, any of the above can be used, regardless of type.

The amount of gelling agent contained in the hard capsule shell of the present invention is not limited, as long as the hard capsule shell can be formed by the cold gelation method. The amount of gelling agent can be, for example, from 0.05 to 2 wt.%, from 0.1 to 1 wt.%, from 0.50-1.5 wt.%, such as of 0.7 wt.%, 0.8 wt.%, 0.9 wt.% or 1.0 wt.%, based on 100 wt.% of water-soluble film forming compound, gelling agent, optionally, the gelling aid, ZnO, and CaCO₃.

A gelling aid can also be used depending on the type of gelling agent used. The gelling aid has an effect of promoting gelation of the gelling agent or may contribute to acceleration of the gelation by directly acting on the cellulose compound to raise or lower the gelation temperature or cloud point temperature. When carrageenan is used as a gelling agent, for example, the following gelling aids can be used in combination with carrageenan. For kappa-carrageenan, examples of usable gelling aids include compounds capable of generating one or more ions selected from sodium, potassium, ammonium, and calcium ions, such as potassium chloride, potassium phosphate, ammonium chloride, ammonium acetate, and calcium chloride. Preferred are compounds capable of generating sodium ions, potassium ions, or calcium ions in aqueous solutions. For iota-carrageenan, examples of usable gelling aids include compounds capable of generating calcium ions in water, such as calcium chloride. When gellan gum is used as a gelling agent, examples of gelling aids that can be used in combination with the gelling agent include compounds capable of generating in water one or more ions selected from sodium, potassium, calcium, and magnesium ions, such as sodium chloride, potassium chloride, calcium chloride, and magnesium sulfate. In addition, citric acid or sodium citrate can also be used as an organic acid or a water-soluble salt thereof.

In an embodiment, optionally in combination with one or more features of the embodiments defined above, the gelling aid is a compound capable of generating sodium ions, potassium ions, or calcium ions in an aqueous solution. Particularly, the gelling aid is potassium chloride.

The amount of gelling aid contained in the hard capsule shell of the present invention can be set according to the amount and type of gelling agent.

In another embodiment, optionally in combination with one or more features of the embodiments defined above, the gelling aid is in an amount from 0.2 wt.% to 1 wt.% based on 100 wt.% of water-soluble film forming compound, optionally, gelling agent, optionally, the gelling aid, ZnO, and CaCO₃. In another embodiment, the gelling aid is in an amount from 0.30 wt.% to 0.7 wt.%, preferably from 0.3 wt.% to 0.4 wt.% based on 100 wt.% of water-soluble film forming compound, optionally, gelling agent, optionally, the gelling aid, ZnO, and CaCO₃.

As mentioned above, TiO₂ free hard capsule shell of the present disclosure comprises ZnO and CaCO₃ wherein ZnO and CaCO₃ are in a suitable amount to provide a white color to the film.

In another embodiment, optionally in combination with one or more features of the embodiments defined above, the ZnO and CaCO₃ together are in an amount from 1 to 20 wt.%, preferably from 2 to 16 wt.%, more preferably from 3 to 14 wt.%, based on 100 wt.% of water-soluble film forming compound, optionally, gelling agent, optionally, the gelling aid, ZnO, and CaCO₃.

In an embodiment, optionally in combination with one or more features of the embodiments defined above, ZnO is in an amount from 33 to 64 wt.% or from 50 to 76 wt.%, based on the total amount of ZnO and CaCO₃.

In an embodiment, optionally in combination with one or more features of the embodiments defined above, the water-soluble film forming compound is a water-soluble cellulose compound and is in an amount from 77.4 to 97.7 wt.%, the gelling agent is in an amount 0.7 to 0.9 wt.%, the gelling aid is in an amount from 0.3 to 0.4 wt.% and the ZnO and CaCO₃ together are in an amount from from 1 to 20 wt.% based on 100 wt.% of water-soluble film forming compound, optionally, gelling agent, optionally, the gelling aid, ZnO, and CaCO₃.

In another embodiment, optionally in combination with one or more features of the embodiments defined above, the water-soluble film forming compound is a water-soluble cellulose compound and is in an amount from 85 to 94 wt.%, the gelling agent is in an amount 0.7 to 0.9 wt.%, the gelling aid is in an amount from 0.3 to 0.4 wt.% and and the ZnO and CaCO₃ together are in an amount from 4.3 to 9.1 wt.% based on 100 wt.% of water-soluble film forming compound, optionally, gelling agent, optionally, the gelling aid, ZnO, and CaCO₃.

In another embodiment, optionally in combination with one or more features of the embodiments defined above, the water-soluble film forming compound is a water-soluble cellulose compound and is in an amount from 87 to 93 wt.%, the gelling agent is in an amount 0.7 to 0.8 wt.%, the gelling aid is in an amount from 0.3 to 0.4 wt.% and the ZnO and CaCO₃ together are in an amount from 2.4 to 9.0 wt.% based on 100 wt.% of water-soluble film forming compound, optionally, gelling agent, optionally, the gelling aid, ZnO, and CaCO₃.

In another embodiment, optionally in combination with one or more features of the embodiments defined above, the water-soluble film forming compound is a water-soluble cellulose compound and is in an amount from 91 to 97 wt.%, the gelling agent is in an amount 0.8 to 0.9 wt.%, the gelling aid is in an amount from 0.3 to 0.4 wt.% and the ZnO and CaCO₃ together are in an amount from 1.7 to 5.0 wt.% based on 100 wt.% of water-soluble film forming compound, optionally, gelling agent, optionally, the gelling aid, ZnO, and CaCO₃.

In an embodiment, optionally in combination with one or more features of the embodiments defined above, the water-soluble cellulose compound is HPMC, the gelling agent is carrageenan, particularly kappa-carrageenan; and the gelling aid is potassium chloride.

In another embodiment, optionally in combination with one or more features of the embodiments defined above, the water-soluble film forming compound is a gelatin and is in an amount from 77 to 98 wt.% and the ZnO and CaCO₃ together are in an amount from 1.2 to 20.3 wt.%, based on 100 wt.% of gelatin, ZnO, and CaCO₃.

In another embodiment, optionally in combination with one or more features of the embodiments defined above, the water-soluble film forming compound is a gelatin and is in an amount from 84 to 94 wt.% and the ZnO and CaCO₃ together are in an amount from 3.0 to 13.6 wt.%, based on 100 wt.% of gelatin, ZnO, and CaCO₃.

In another embodiment, optionally in combination with one or more features of the embodiments defined above, the water-soluble film forming compound is a gelatin and is in an amount from 86 to 94 wt.% and the ZnO and CaCO₃ together are in an amount from 5.7 to 11.6 wt.%, based on 100 wt.% of gelatin, ZnO, and CaCO₃.

In another embodiment, optionally in combination with one or more features of the embodiments defined above, the water-soluble film forming compound is a gelatin and is in an amount from 92 to 98 wt.% and the ZnO and CaCO₃ together are in an amount from 1.2 to 6.8 wt.%, based on 100 wt.% of gelatin, ZnO, and CaCO₃.

In an embodiment, optionally in combination with one or more features of the embodiments defined above, the film further comprises starch.

In another embodiment, optionally in combination with one or more features of the embodiments defined above, the starch is in an amount from 1 wt.% to 34 wt.%, particularly from 5 wt.% to 25 wt.%, based on 100 wt.% of the total amount of ZnO, CaCO₃, and starch. In another embodiment, the starch is in an amount from 10 wt.% to 20 wt.% based on 100 wt.% of the total amount of ZnO, CaCO₃, and starch.

The hard capsule capsule shell of the present disclosure can also comprise a plasticizer, a lubricant, a sequestrant, an additional colorant such as a dye or a pigment, and a light-shielding agent.

Any plasticizer can be used without limitation, as long as it can be used for food compositions. Examples of plasticizers include dioctyl adipate, adipic acid polyester, epoxidated soybean oil, diester of epoxyhexahydrophthalic acid, kaolin, triethyl citrate, glycerol, glycerol fatty acid ester, sesame oil, a mixture of dimethylpolysiloxane and silicon dioxide, D-sorbitol, medium-chain triglyceride, corn starch-derived liquid sugar alcohol, triacetin, concentrated glycerin, castor oil, phytosterol, diethyl phthalate, dioctyl phthalate, dibutyl phthalate, butyl phthalyl butyl glycolate, propylene glycol, polyoxyethylene (105) polyoxypropylene (5) glycol, Polysorbate 80, Macrogol 1500, Macrogol 400, Macrogol 4000, Macrogol 600, Macrogol 6000, isopropyl myristate, a mixture of cottonseed oil and soybean oil, glyceryl monostearate, isopropyl linoleate, and the like. When a plasticizer is used, the plasticizer can be usually added, for example, in an amount of 8.6 wt.% or less, such as of 4.3 wt.%, 2.5 wt.%, or 2.1 wt.%, based on 100 wt.% of the total hard capsule components, excluding moisture.

Examples of sequestrants include ethylenediaminetetraacetic acid, acetic acid, boric acid, citric acid, gluconic acid, lactic acid, phosphoric acid, tartaric acid, or salts of these acids, metaphosphate, dihydroxyethylglycine, lecithin, β-cyciodextrin, and combinations thereof.

Any lubricant can be used without limitation, as long as it can be used or food compositions. Examples of lubricants include calcium stearate, magnesium stearate, sodium stearyl fumarate, carnauba wax, starch, sucrose fatty acid ester, light anhydrous silicic acid, macrogol, talc, hydrogenated vegetable oil, and the like.

Any additional colorant and any light-shielding agent can be used without limitation, as long as they can be used food compositions. Examples of additoinal colorants include powdered gambir tannin, turmeric extract, methylrosanilinium chloride, yellow iron oxide, yellow iron sesquioxide, OPASPRAY K-1-24904, orange essence, brown iron oxide, carbon black, caramel, carmine, carotene liquid, β-carotene, light-sensitive element No. 201, licorice extract, gold leaf, Sasa albomarginata extract, black iron oxide, light anhydrous silicic acid, Daemonorops draco (kekketsu ), zinc oxide, iron sesquioxide, disazo yellow, Food Blue No. 1 and its aluminum lake, Food Blue No. 2 and its aluminum lake, Food Yellow No. 4 and its aluminum lake, Food Yellow No. 5 and its aluminum lake, Food Green No. 3 and its aluminum lake, Food Red No. 2 and its aluminum lake, Food Red No. 3 and its aluminum lake, Food Red No. 102 and its aluminum lake, Food Red No. 104 and its aluminum lake, Food Red No. 105 and its aluminum lake, Food Red No. 106 and its aluminum lake, sodium hydroxide, talc, sodium copper chlorophyllin, copper chlorophyll, powdered hull-less barley green tea extract, hull-less barley green tea extract, phenol red, sodium fluorescein, d-borneol, malachite green, octyldodecyl myristate, methylene blue, medicinal carbon, riboflavin butyrate, riboflavin, powdered green tea, manganese ammonium phosphate, riboflavin sodium phosphate, rose oil, turmeric color, chlorophyll, carminic acid color, Food Red No. 40 and its aluminum lake, water-soluble annatto, sodium iron chlorophyllin, dunaliella carotene, paprika color, carrot carotene, potassium norbixin, sodium norbixin, palm oil carotene, beat red, grape pericarp color, black currant color, monascus color, safflower red color, safflower yellow color, marigold color, sodium riboflavin phosphate, madder color, alkanet color, aluminum, potato carotene, shrimp color, krill color, orange color, cacao color, cacao carbon black, oyster color, crab color, carob color, fish scale foil, silver, kusagi (Clerodendrum trichotomum ) color, gardenia blue color, gardenia red color, gardenia yellow color, kooroo color, chlorophin, kaoliang color, bone carbon black, bamboo grass color, shea nut color, lithospermum root color, red sandalwood color, vegetable carbon black, sappan color, spirulina color, onion color, tamarind color, corn color, tomato color, peanut color, phaffia color, pecan nut color, monascus yellow, powdered annatto, haematococcus algae color, purple sweet potato color, purple corn color, purple yam color, vegetable oil soot color, lac color, rutin, enju (Styphnolobium japonicum ) extract, buckwheat whole-plant extract, logwood color, red cabbage color, red rice color, red radish color, adzuki bean color, Hydrangea serrata leaf extract, sepia color, uguisukagura (Lonicera gracilipes ) color, elderberry color, olive tea, cowberry color, gooseberry color, cranberry color, salmon berry color, strawberry color, dark sweet cherry color, cherry color, thimbleberry color, deberry color, pineapple juice, huckleberry color, grape juice color, black currant color, blackberry color, plum color, blueberry color, berry juice, boysenberry color, whortleberry color, mulberry color, morello cherry color, raspberry color, red currant color, lemon juice, loganberry color, chlorella powder, cocoa, saffron color, beefsteak plant color, chicory color, laver color, hibiscus color, malt extract, paprika powder, red beet juice, carrot juice, and the like.

Examples of light-shielding agents include iron sesquioxide, yellow iron sesquioxide, black iron oxide, Food Blue No. 1 aluminium lake, Food Blue No. 2 aluminium lake, Food Yellow No. 4 aluminium lake, Food Yellow No. 5 aluminium lake, Food Green No. 3 aluminium lake, Food Red No. 2 aluminium lake, Food Red No. 3 aluminum lake, Food Red No. 102 aluminium lake, Food Red No. 104 aluminium lake, Food Red No. 105 aluminium lake, Food Red No. 1 06 aluminium lake, Red No. 40 aluminium lake, and the like.

In general, the capsule film after preparation preferably contains a small percentage of residual moisture. When capsule shells after molding are dried at a temperature in the range of 30 °C to 100 °C, the moisture content of the capsule shells settles to a specific saturated residual moisture level according to the solids content and the composition of the capsule shells. Naturally, when the drying treatment is performed at a higher temperature, the moisture content settles to a saturated moisture level in a shorter period of time. The residual moisture content depends on environmental humidity during capsule storage, and changes almost reversibly. That is, the saturated moisture level of the capsule film after fully drying at 30 to 100 °C settles to a constant value, if the film is further stored at a constant temperature and relative humidity for several days after the drying.

Containing a small amount of residual moisture is rather preferable in order to maintain breakage resistance. The residual moisture content, as measured at room temperature and a relative humidity of 43% in terms of saturated moisture level, can be at least 1.5% or more, particularly from 1.5% to 14.5%, based on the total weight of the hard capsule shell.

The residual saturated moisture level can be expressed in terms of water content calculated from loss-on-drying. Water content of capsule films or capsule shells can be determined by standard methods known by those skilled in the art.

### Hard Capsule shell-Preparing suspension

As mentioned above, the suspension for preparing the TiO₂ free hard capsule shell of the present disclosure (the "hard capsule shell-preparing suspension") is a TiO₂ free suspension comprising an aqueous solvent and the hard capsule shell components described above, that is a water-soluble film forming compound, a gelling agent, optionally, a gelling aid, ZnO and CaCO₃, wherein the suspension is for the preparation of a hard capsule shell comprising a film, and ZnO and CaCO₃ are in a suitable amount to provide a white color to the film. Examples of aqueous solvents include water, and a mixture of ethanol and water. Particularly, the solvent is water.

The amounts of the components contained in the hard capsule shell-preparing suspension are not limited, as long as the hard capsule shell can be formed by the cold gelation method. The amounts of water-soluble film forming compound, of gelling agent, of gelling aid, and of ZnO and CaCO₃, are the same as defined above for the hard capsule shell components.

The concentration of the total components in the hard capsule shell-preparing suspension or solution can be, for example, from 20 to 37 wt.%. In an embodiment, the water-soluble film forming compound is a water soluble cellulose compound and the concentration of the total components in the hard capsule shell-preparing suspension is from 20 to 22 wt.%. In an embodiment, the water-soluble film forming compound is gelatin and the concentration of the total components in the hard capsule shell-preparing solution is from 33 to 37 wt.%.

### General method for preparing the hard capsule shell

The hard capsule shells of the present disclosure can be prepared as follows. First, the water-soluble film forming compound, such as the water-soluble cellulose compound, is dispersed in hot water at about 70 to 90 °C and, then the dispersion can be cooled to dissolve the water-soluble film forming compound. The resulting suspension can be heated again, for instance, to about 30 to 60 °C, and, optionally, a gelling agent and, optionally, a gelling aid are added to and dispersed and/or dissolved in the suspension to prepare a uniform capsule shell-preparing suspension (immersion liquid), followed by adjusting the solution to a desired temperature of immersion liquid. A dispersion of a colorant can be added either to the first dispersion or to the final suspension.

Thus, it also forms part of the invention a method for preparing a capsule-preparing suspension comprising the step of preparing a first suspension of a water-soluble film forming compound, optionally, adding a gelling agent and, optionally, a gelling aid; and adding a dispersion comprising ZnO and CaCO₃ to the first suspension. When the water-soluble film forming compound is a water-soluble cellulose, a gellling agent and, optionally, a gelling aid are added.

In an embodiment, optionally in combination with one or more features of the embodiments defined above, in the method above, ZnO and CaCO₃ are added in the form of a powder comprising a mixture of ZnO, CaCO₃, and starch. More particularly, ZnO and CaCO₃ are added in the form of a powder consisting of a mixture of ZnO, CaCO₃, and starch.

In a particular embodiment, optionally in combination with one or more features of the embodiments defined above, the powder consists of a mixture of 30 to 76 wt.% ZnO, 20 to 50 wt.% CaCO₃, and 1 to 34 wt.% starch, or of 33 to 60 wt.% ZnO, 20 to 33 wt.% CaCO₃, and 10 to 34 wt.% starch. In an embodiment the powder consists of a mixture of 33 wt.% ZnO, 33 wt.% CaCO₃, and 34 wt.% starch. In another embodiment the powder consists of a mixture of 60 wt.% ZnO, 20 wt.% CaCO₃, and 20 wt.% starch. In another embodiment the powder consists of a mixture of 46 wt.% ZnO, 40 wt.% CaCO₃, and 14 wt.% starch.

The incorporation of ZnO and CaCO₃ in the form of such a powder allows obtaining a hard capsule shell wherein ZnO and CaCO₃. are distributed more homogeneously than TiO₂, when using the later as colorant instead.

It also forms part of the invention a method for preparing a hard capsule shell, the method comprising the step of preparing a hard capsule film using the hard capsule shell-preparing suspension as defined herein above. Particularly, the method is a cold gelation method, i.e. the hard capsule shell is produced by means of dip molding.

An inherent result of the method of the invention is that it allows obtaining a hard capsule shell having improved properties compared with the one of the hard capsule shells of the prior art. Therefore, a hard capsule shell obtainable by the method defined above is also part of the invention.

In the present disclosure, it is noted that when discussing the hard capsule shell composition, the hard capsule shell-preparing suspension, and the method of preparing the hard capsule shell, each one of the embodiments or features defined for any of the mentioned aspects can be considered applicable to each one of the other aspects, whether or not they are explicitly discussed in the context of that other aspect. Thus, for example, when defining embodiments of the hard capsule shell *per se*, such embodiments also refer to the hard capsule shell-preparing suspension, (and to the method of preparing the hard capsule shell), and *vice versa*.

The viscosity of the capsule-preparing solution is not particularly limited. Preferably, the viscosity of the capsule-preparing solution can be adjusted to 100 to 20,000 mPa·s, and more preferably 300 to 10,000 mPa·s, under the temperature conditions used for immersion of a capsule-forming pin (temperature of immersion liquid) (30 to 80 °C, preferably 40 to 60 °C). The solvent content of the capsule-preparing solution can be, for example, typically from 60 to 90 wt.%, particularly from 67 to 85 wt.%. More particularly, when the water-soluble film forming compound is a water-soluble cellulose compound the solvent content of the capsule-preparing solution can be from 78 to 80 wt.%, and when it is gelatin the the solvent content of the capsule-preparing solution can be from from 63 to 70 wt.%.

The viscosity herein refers to a viscosity as measured with a Brookfield rotational viscometer at a predetermined temperature at 60 rpm for 1 minute using a No. 2 rotor for a viscosity of less than 500 mPa·s, a No. 3 rotor for a viscosity of at least 500 mPa·s and less than 2000 mPa·s, and a No. 4 rotor for a viscosity of 2000 mPa·s or more. The concentration of each component contained in the capsule-preparing solution is described below.

The method for preparing (molding) a hard capsule shell is not particularly limited, as long as the capsule-preparing suspension according to the present invention is used to prepare a capsule. A hard capsule shell is generally produced by immersing a mold pin, which is a mold for forming capsule shells, into an aqueous solution of capsule film-forming components; then curing and drying the film adhering to the mold pin when the mold pin is withdrawn from the solution, to thereby obtain a capsule with a desired shape and thickness (the dipping method). Specifically, the method for preparing a hard capsule shell may comprise the steps of: preparing a hard capsule shell-preparing suspension by the method above; and dipping a capsule-forming pin into the hard capsule shell-preparing solution and then withdrawing the pin from the solution to allow the solution adhering to the capsule-forming pin to gel, followed by drying the gelled film at 20 to 80 °C to prepare a capsule shell. More specifically, the hard capsule shell of the present disclosure can be produced through the following molding steps:
- (1) a step of immersing a capsule-forming pin into a hard capsule shell-preparing suspension (immersion liquid) containing a film forming compound, optionally, a gelling agent, optionally, a gelling aid, and a white colorant comprising ZnO and CaCO₃ (dipping step);
- (2) a step of withdrawing the capsule-forming pin from the hard capsule shell-preparing solution (immersion liquid) to allow the solution adhering to the outer surface of the capsule-forming pin to gel (gelation step);
- (3) a step of drying the gelled capsule film (gelled film) formed on the outer surface of the capsule-forming pin (drying step);
- (4) a step of removing the dried capsule film from the capsule-forming pin (removal step), and
- (5) optionally, if necessary, a step of heating the gelled capsule film (gelled film) to 20 to 80 °C, which is performed after the gelation step (2) and which can be before, after, or simultaneously with the drying step (3) or after the removal step (4).

The capsule film thus prepared is cut to a predetermined length, and then provided as a hard capsule shell with a pair of a body portion and a cap portion being engaged with each other.

The film thickness of hard capsule shells is usually in the range of 50 to 200 µm.

As mentioned above, the present disclosure also relates to a capsule product comprising a hard capsule shell as defined herein filled with a formulation comprising an active ingredient. The content encapsulated in the hard capsule shell is not particularly limited. Examples of active ingredient include, but are not limited to, an active pharmaceutical ingredient for humans and animals, a nutritional supplement, a nutraceuticals, a cosmetic, a health food, a vitamin, a mineral, and mixtures thereof. The form of the content is also not particularly limited. For example, the content can be in the form of a liquid, gel, powder, granules, tablets, pellets, or a mixture thereof (a hybridized state).

Throughout the description and claims the word "comprise" and variations of the word, are not intended to exclude other technical features, additives, components, or steps. Furthermore, the word "comprise" encompasses the case of "consisting of".

The following examples and drawings are provided by way of illustration, and they are not intended to be limiting of the present invention. Furthermore, the present invention covers all possible combinations of particular and preferred embodiments described herein.

### Examples

### EXAMPLE 1 AND 2 AND COMPARATIVE EXAMPLES 1 AND 2

TiO₂ free capsule shells according to the present disclosure were prepared (Example 1 and 2) and, additionally, as comparative examples, a capsule shell without colorant (Comparative Example 1) and another one wherein the colorant/opacifier used was TiO₂ (Comparative Example 2).

Various tests were carried out to determine the differences and similarities between the the three capsule shells.

### 1.1. Description of the capsule shells

Several hard capsule shells with or without colorant having the compositions shown in Table 1 were prepared.

**Table 1**

| **wt.% of component** | **HPMC** | **Gelling agent (κ-Carrageenan)** | **Gelling aid (KCl)** | **Water** | **Colorant** | **Total** |
|---|---|---|---|---|---|---|
| Comp. Ex. 1 | 93.77 | 0.85 | 0.38 | 5 | 0 | 100 |
| Comp. Ex. 2 | 91.79 | 0.83 | 0.37 | 5 | 2⁽¹⁾ | 100 |
| Example 1 | 88.83 | 0.81 | 0.36 | 5 | 5⁽²⁾ | 100 |
| Example 2 | 83.90 | 0.76 | 0.34 | 5 | 10⁽²⁾ | 100 |

HPMC: hydroxypropyl methylcellulose. Particularly, the HPMC was a mixture of 79% hypromellose with substitution grade 2910 and viscosity type 6 mPa.s (specification 4.8-7.2 mPa.s), and 21% is hypromellose with a substitution grade 2208 and a viscosity range from 3.2 to 4.8 mPa.s.
⁽¹⁾TiO₂ was used as colorant in a 26% TiO₂ water suspension ;
⁽²⁾a free TiO₂ colorant in the form of a 30% wt. water suspension containing a powder consisting of 46 wt.% ZnO, 40 wt.% CaCO₃ and 14 wt.% rice starch (herein below referred as "ONFA" colorant) was used as colorant; CaCO₃ was PPC having a median particle size of 1.0 - 1.8 µm.

### 1.2. Homogeneity of the hard capsule surface

To compare the homogeneity of the surface of the capsule shells prepared with the formulation of Example 1 and with the formulations of Comparative Example 1 and of Comparative Example 2, films with the same formulation as the corresponding capsule shells were prepared and studied using a Field Emission Scanning Electron Microscope (SEM) Jeol JSM-6335F by applying an accelerating voltage of 20 kV or of 5.0 kV, a working distance of 15.0 mm.

The following samples were studied:
- zinc oxide (ZnO) powder;
- calcium carbonate (CaCO₃) powder;
- rice starch powder;
- a TiO₂ 26 wt.% color suspension;
- a film prepared with the composition of Comparative Example 1 ("Film of Comparative Example 1"), i.e. without colorant;
- a film prepared with the composition of Example 1 ("Film of Example 1"), i.e. with the ONFA colorant; and
- a film prepared with the composition of Comparative Example 2 ("TiO₂ film"), i.e. with TiO₂ as colorant.

The samples were treated with graphite evaporation, which allows smooth surfaces to be seen without alterations (optimal treatment for films).

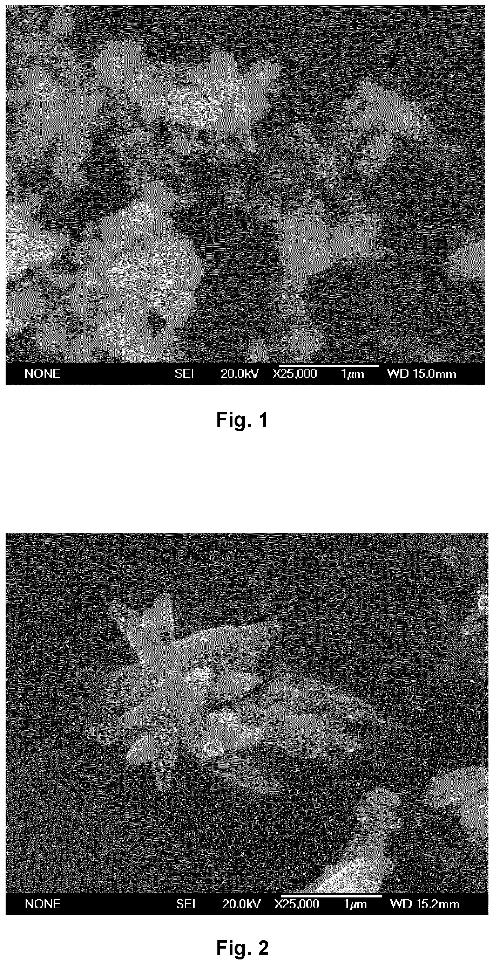

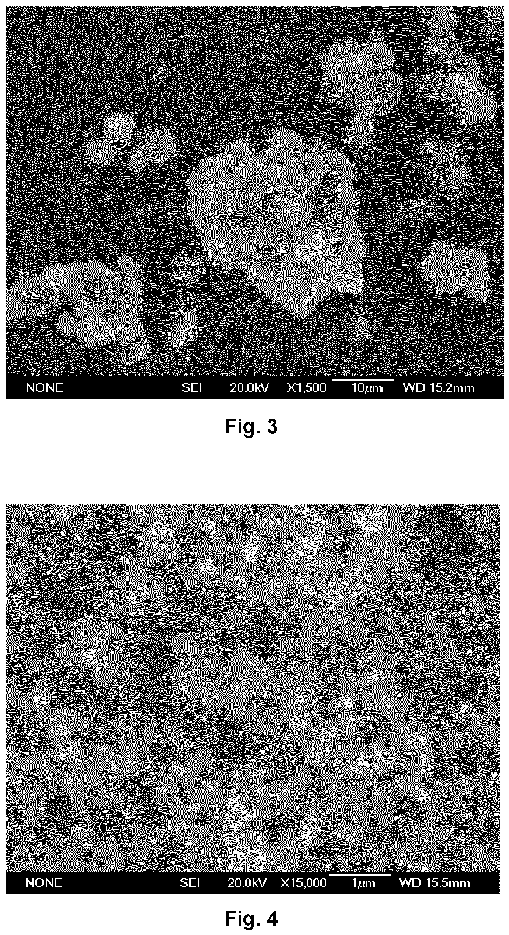

The colorant raw materials, ZnO (Fig. 1), CaCO₃ (Fig. 2) and rice starch (Fig. 3), as well as TiO₂ (Fig. 4) have characteristic structures that allow them to be identified in the electron microscope.

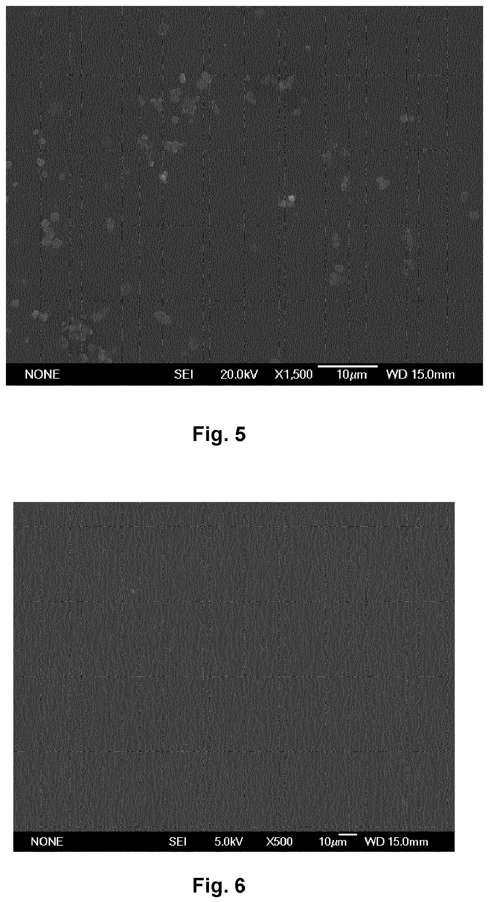

As expected, in the film of Comparative Example 1 some particles can be seen on the surface (Fig. 5), which, according to the elemental analysis, were of KCI, as shown in Table 2.

**Table 2**

| Spectrum | In stats. | C | Cl | K | O | Total |
|---|---|---|---|---|---|---|
| 2-3 Spectrum 1 | Yes | 26.50 | 1.50 | 1.17 | 70.83 | 100.00 |
| 2-3 Spectrum 2 | Yes | 27.23 | 0.24 | | 72.53 | 100.00 |
| Max. | | 27,23 | 1.50 | 1.17 | 72.53 | |
| Min. | | 26.50 | 0.24 | 1.17 | 70.83 | |

| | | | | | | |
|---|---|---|---|---|---|---|
| Processing option: Oxygen by stoichiometry (Normalised) | | | | | | |

All the results are in weight %.

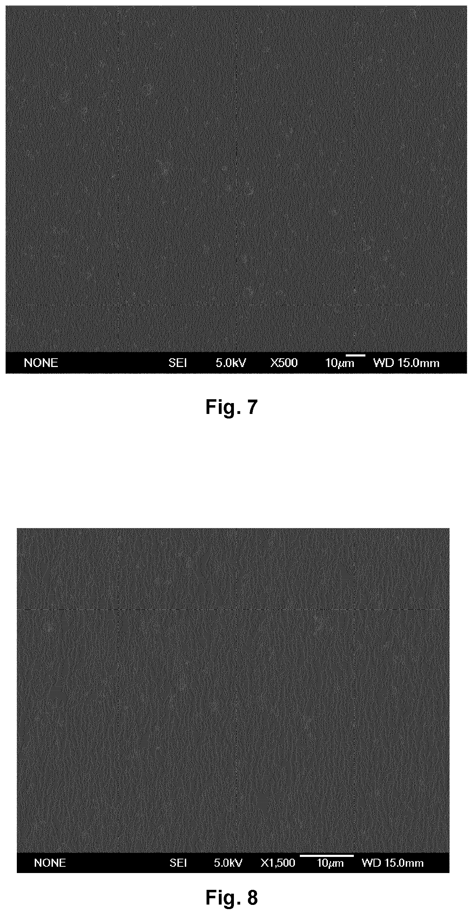

When the colored films at 500 magnifications were compared, in the TiO₂ film (see Fig. 6, at 500x) the observed particles of the colorant were of much smaller size, giving a homogeneous appearance. However, the film of Example 1 presented thicker or agglomerated particles of similar appearance (see Fig. 7, at 500x).

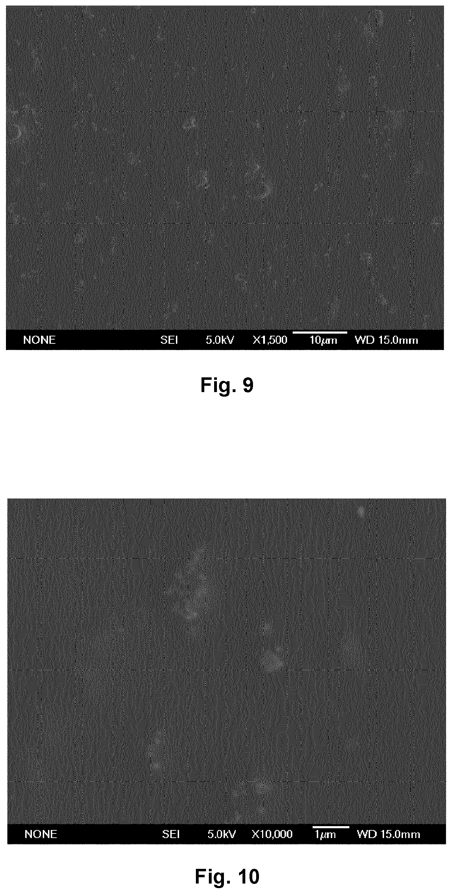

When observed at 1,500 magnifications, the details were better appreciated, that is a greater homogeneity in the distribution of TiO₂ as the colorant agent in the film with the formulation of Comparative Example 2 (Fig. 8) but less presence of agglomerates in the Film of Example 1 (Fig. 9).

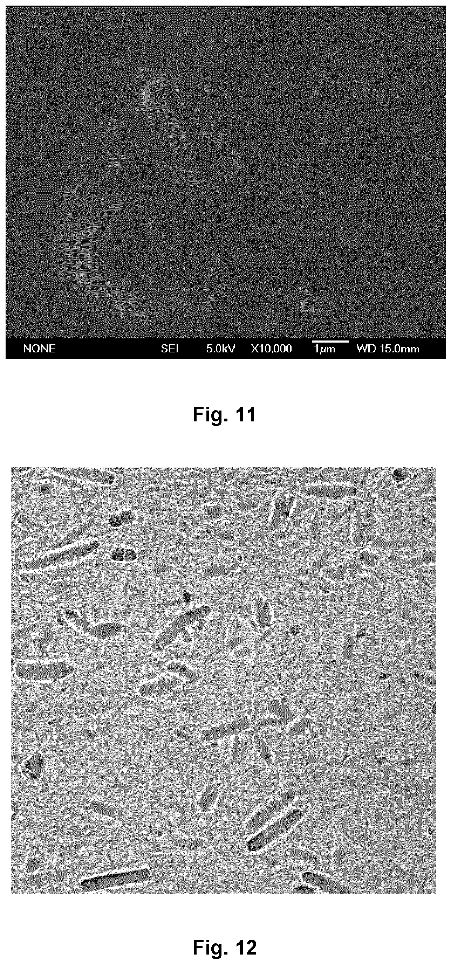

When comparing at 10,000 magnifications (Figs. 10 and 11), it was observed that the shape of the particles found in the film of Example 1 was similar and around 2-3 µm, while the particles of the TiO₂ film were of a diameter lower than 1 µm.

### 1.3. Homogeneity of the colorant in the structure of the hard capsule

To compare the homogeneity in the distribution of the colorant in the structure of the capsule, films with the same formulation as the corresponding capsule shells were prepared and then studied by means of a fluorescence Zeiss Axiopland 2 microscope, with a Qlmaging Retiga 4000R camera and MetaMorph 6.2 software from Universal Imagin corporation.

The following samples were studied:
- film of Comparative Example 1 (without colorant);
- film of Example 1 (with the ONFA colorant); and
- film of Comparative Example 2 ("TiO₂ film" i.e. with TiO₂ as colorant).

It was observed that in films of Comparative example 1 the characteristic fibers of cellulose formed a continuous surface with droplets of gel (Fig. 12).

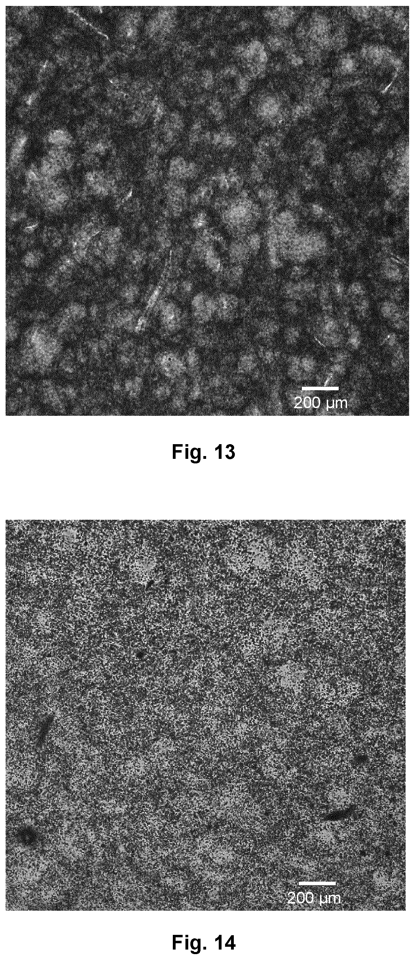

When comparing the TiO₂ film with the film of Example 1, it was observed that in the former (Fig. 13), the colorant was distributed around the cellulose fibers and inside the gel droplets, while in the film of Example 1 (Fig. 14) the colorant was distributed more homogeneously, that is, the colorant was distributed both inside the droplets and the rest of the film, better covering the entire surface.

Without wishing to be bound by theory, it is thought that this could explain the improvement in the strength and flexibility of the film of Example 1.

### 1.4. Elongation at break

In order to compare the elongation at break of the different samples, films made with the same formulation as the corresponding capsule shells were tested in a TA.XTPlus Texture Analyser with the following conditions:
- Probe: Tension tweezers with non-slip coating (Ref. A/TGR)
- Test Mode: Traction
- Sequence title: Back to top
- Pre-test speed: 1 mm/s
- Test speed: 1 mm/s
- Post-test speed: 10mm/s
- Activation force: Auto - 15 gF
- Apply: Distance
- Distance to travel: 10 mm
- Load cell: 30 kgF
- Data acquisition rate: 500 pps

The specimens measured had an average thickness of 0.111 mm and a relative humidity between 3.7 and 4.2%.

Films of Example 1 and TiO₂ films were tested. As will be seen from Tables 3 and 4 below, better results were obtained with the films of Example 1 (TiO₂ free, Table 3) than with the TiO₂ films (Table 4). In Tables 3 and 4, S stands for standard deviation and RSD for relative standard deviation.

The films of Example 1 showed a final force (10.031.42 ± 246.38 g) similar to the TiO₂ films (10.162.87 ± 240.66 g), while the elongation results were higher on the films of Example 1 (3.35 ± 0.56 mm) than on the TiO₂ films (2.14 ± 0.51 mm), which means that the film of Example 1 is more elastic and, consequently, is less likely to break when subjected to the same load, than the TiO₂ film.

**Table 3 - Elongation at break of films of Example 1**

| **ID Assay** | **Initial force (g)** | **Initial distance (mm)** | **Initial Stress (kg/mm²)** | **Initial deformation (%)** | **Final force (g)** | **Final distance (mm)** | **Final stress (kg/mm²)** | **Final deformation (%)** |
|---|---|---|---|---|---|---|---|---|
| 1 | 9649.0 | 2.43 | 6.84 | 8.11 | 10241.5 | 6.26 | 7.26 | 20.86 |
| 2 | 9690.4 | 2.36 | 7.00 | 7.87 | 10272.0 | 6.50 | 7.42 | 21.66 |
| 3 | 9733.8 | 2.57 | 6.92 | 8.58 | 10397.7 | 6.38 | 7.40 | 21.26 |
| 4 | 9614.4 | 2.37 | 6.83 | 7.89 | 9955.2 | 5.74 | 7.07 | 19.13 |
| 5 | 9516.1 | 2.28 | 6.88 | 7.60 | 9941.1 | 5.76 | 7.19 | 19.21 |
| 6 | 9379.4 | 2.30 | 6.91 | 7.65 | 9944.1 | 6.06 | 7.32 | 20.18 |
| 7 | 9707.1 | 2.49 | 7.05 | 8.29 | 10102.7 | 5.63 | 7.34 | 18.77 |
| 8 | 9809.1 | 2.57 | 7.06 | 8.58 | 10110.9 | 5.31 | 7.28 | 17.70 |
| 9 | 9395.7 | 2.49 | 6.87 | 8.31 | 9568.7 | 5.13 | 7.00 | 17.09 |
| 10 | 9601.7 | 2.46 | 6.68 | 8.20 | 9780.3 | 5.02 | 6.81 | 16.72 |
| **Total Average:** | **9609.67** | **2.43** | **6.90** | **8.11** | **10031.42** | **5.78** | **7.21** | **19.26** |
| S | 141.57 | 0.10 | 0.11 | 0.35 | 246.38 | 0.52 | 0.19 | 1.73 |
| RSD | 1.47 | 4.30 | 1.64 | 4.30 | 2.46 | 9.00 | 2.70 | 9.00 |

**Table 4 - Elongation at break of TiO₂ films**

| **ID Assay** | **Initial force (g)** | **Initial distance (mm)** | **Initial Stress (kg/mm²)** | **Initial deformation (%)** | **Final force (g)** | **Final distance (mm)** | **Final stress (kg/mm²)** | **Final deformation (%)** |
|---|---|---|---|---|---|---|---|---|
| 1 | 10273.4 | 2.46 | 7.34 | 8.20 | 10436.2 | 4.56 | 7.46 | 15.19 |
| 2 | 9690.6 | 2.38 | 7.16 | 7.92 | 10098.6 | 5.01 | 7.47 | 16.69 |
| 3 | 9677.6 | 2.51 | 7.26 | 8.37 | 9953.2 | 5.08 | 7.47 | 16.94 |
| 4 | 10096.0 | 2.44 | 7.29 | 8.14 | 10423.5 | 5.05 | 7.52 | 16.83 |
| 5 | 9801.0 | 2.55 | 7.13 | 8.49 | 10098.3 | 5.09 | 7.34 | 16.95 |
| 6 | 10049.3 | 2.819 | 7.193 | 9.397 | 10461.0 | 5.439 | 7.488 | 18.130 |
| 7 | 9658.5 | 2.341 | 7.141 | 7.803 | 9775.9 | 4.139 | 7.228 | 13.797 |
| 8 | 10154.8 | 2.275 | 7.459 | 7.583 | 10348.5 | 3.947 | 7.601 | 13.157 |
| 9 | 9968.1 | 2.391 | 7.504 | 7.970 | 10089.4 | 3.875 | 7.595 | 12.917 |
| 10 | 9878.6 | 2.407 | 7.196 | 8.023 | 9944.1 | 3.785 | 7.243 | 12.617 |
| **Total Average:** | **9924.79** | **2.46** | **7.27** | **8.19** | **10162.87** | **4.60** | **7.44** | **15.32** |
| S | 217.42 | 0.15 | 0.13 | 0.50 | 240.66 | 0.61 | 0.13 | 2.04 |
| RSD | 2.19 | 6.11 | 1.81 | 6.11 | 2.37 | 13.31 | 1.76 | 13.31 |

### 1.5. Capsule rupture force

Using the same TA.XTPlus Texture Analyser, with a TA-CLT accessory to pull the capsule, every part of the capsule itself can be compared between the different samples.

The TA-CLT accessory (Capsule Loop Tensile Test Fixture) is used to measure the force required to split one half of a hard gel capsule.

The test conditions were:
- Probe: Capsule accessory (Ref. TA-CLT)
- Test Mode: Traction
- Sequence title: Back to top
- Pre-test speed: 1 mm/s
- Test speed: 1 mm/s
- Post-test speed: 10mm/s
- Activation force: 10 g
- Apply: Distance
- Distance to travel: 5 mm
- Load cell: 30 kgF
- Data acquisition rate: 1000 pps

The white capsule shells tested had an average relative humidity from 4.2 to 4.5%, while the average thickness was 0.102 mm.

Capsule shells were prepared with the composition of Example 1 ("capsule shells of Example 1") and with the composition of Comparative Example 2 ("TiO₂ capsule shells")

As can be seen from Tables 5 and 6, the best results were obtained from the analysis of the capsule shells of Example 1, whose cap broke at a final force of 9191.2 ± 459.7 g, elongating an average of 0.510 ± 0.097 mm, namely they have a greater elongation than TiO₂ capsule shells. In conclusion, capsule shells of Example 1 were less fragile and more elastic than TiO₂ capsule shells.

**Table 5 - Results of capsule shells of Example 1**

| **ID Assay-Capsules** | **Initial force** | **Initial distance** | **Final force** | **Final distance** |
|---|---|---|---|---|
| **Example 1** | **9** | **mm** | **g** | **mm** |
| 1 | 7753.4 | 0.844 | 9538.6 | 1.366 |
| 2 | 7671.3 | 0.827 | 9362.6 | 1.459 |
| 3 | 7597.4 | 0.845 | 9334.5 | 1.381 |
| 4 | 8268.6 | 0.879 | 9873.2 | 1.469 |
| 5 | 7303.7 | 0.822 | 8719.7 | 1.356 |
| 6 | 7954.5 | 0.845 | 9673.4 | 1.475 |
| 7 | 7270.8 | 0.839 | 8390.7 | 1.217 |
| 8 | 7489.8 | 0.813 | 8855.4 | 1.183 |
| 9 | 7565.5 | 0.828 | 8951.8 | 1.234 |
| 10 | 7199.2 | 0.836 | 9211.9 | 1.336 |
| **Total Average:** | **7607.4** | **0.838** | **9191.2** | **1.348** |
| S | 328.4 | 0.018 | 459.7 | 0.107 |
| RSD | 4.32 | 2.15 | 5.00 | 7.90 |

**Table 6 - Results of TiO₂ containing capsule shells**

| **ID Assay - Capsules** | **Initial force** | **Initial distance** | **Final force** | **Final distance** |
|---|---|---|---|---|
| **Comp. Ex. 2** | **g** | **mm** | **g** | **mm** |
| 1 | 8978.8 | 0.947 | 10573.2 | 1.377 |
| 2 | 9220.2 | 0.941 | 11576.7 | 1.577 |
| 3 | 9138.1 | 0.949 | 9903.6 | 1.245 |
| 4 | 9142.5 | 0.939 | 9811.8 | 1.201 |
| 5 | 9223.1 | 0.939 | 10079.7 | 1.199 |
| 6 | 9338.4 | 0.959 | 11257.3 | 1.495 |
| 7 | 8804.9 | 0.940 | 9169.3 | 0.988 |
| 8 | 8780.4 | 0.936 | 10861.1 | 1.714 |
| 9 | 9538.5 | 0.945 | 10645.4 | 1.181 |
| 10 | 9264.5 | 0.940 | 9587.3 | 1.000 |
| **Total Average:** | 9142.9 | 0.9 | 10346.5 | 1.298 |
| S | 234.4 | 0.0 | 764.8 | 0.240 |
| RSD | 2.56 | 0.72 | 7.39 | 18.46 |

### 1.6. Nutrients

As explained above, the white composition of Example 1 contains the ONFA colorant comprising calcium carbonate and zinc oxide. According to the Food and Agriculture Organization (FAO), calcium and zinc are essential nutrients being among the most important minerals in human nutrition. For this reason, the TiO₂ free capsule shells of the invention, comprising the composition of Example 1, provide the additional advantage, over the capsule shells wherein TiO₂ is used as a white colorant, of providing the mentioned essential nutrients.

### 1.7. Color/Opacity

As far as colors are concerned, the films of Example 1 and 2 were compared with the TiO₂ film of Comparative Example 2.

Table 7 shows the opacity and the CIELAB color space (*L***a***b***,* measured with a LabScan^{®} XE from HunterLab) of the film of Example 1 and a TiO₂ film, wherein *L** is the lightness value and defines black at 0 and white at 100; the a* axis is relative to the green-red opponent colors, with negative values toward green and positive values toward red; and the b* axis represents the blue-yellow opponents, with negative numbers toward blue and positive toward yellow.

**Table 7**

| Color comparation | *L** | *a** | *b** | Opacity |
|---|---|---|---|---|
| Film of Comp. Ex. 2 (containing 2% of TiO₂) | 94,18 | -0,69 | 6,32 | 60,7 |
| Film of Ex. 1 (containing 5% of the ONFA colorant) | 94,44 | -0,91 | 5,23 | 40,9 |
| Film of Ex. 2 (containing 10% of the ONFA colorant) | 94,63 | -0,54 | 6,79 | 52,8 |

As can be seen, the films of Examples 1 and 2 had a higher lightness ("whiter") while having an acceptable opacity than the TiO₂ film of Comparative Example 2.

### 1.8. pH

The pH's of the white color suspensions mentioned below and their influence in the white colored final cellulose suspension is shown in Table 8.

The pH of the ONFA white color suspension (30 wt.%), containing ZnO and CaCO₃, is 11. This is almost 4 points more basic than that of the 26 wt.% TiO₂ suspension, which is almost neutral (pH = 6.8).

However, the uncolored cellulose suspension has a pH of 7.4, which in the case of the cellulose suspension containing a 2 wt.% TiO₂ falls to 6.9 due to the effect of the white colorant. Nevertheless, in the cellulose suspension containing the ONFA white colorant (which is TiO₂ free), even though the white colorant is at 5 wt.% concentration, the pH appears to be less affected by the presence of colorant.

**Table 8**

| **Sample** | **pH1** | **pH2** | **Media** |
|---|---|---|---|
| Colored suspension TiO₂ | 6.80 | 6.72 | 6.76 |
| Colored suspension with ONFA | 10.98 | 11.02 | 11.00 |
| Comp. Ex. 1 | 7.39 | 7.40 | 7.40 |
| Comp. Ex. 2 (2%) | 6.94 | 6.93 | 6.93 |
| Example 1 (5%) | 7.74 | 7.77 | 7.76 |

It is known that bacteria are sensitive to various pH ranges. Bacteria show the best growth at optimum pH, i.e., 6.5 - 7.5. However, the bacterial species that grow at a pH range above or below the preferred value would not survive.

The acidic pH usually restricts microbial growth, and it is frequently used in food preservation methods. But the basic pH also inhibits microbial growth, for this reason, the formulation of Example 1 comprising a mixture of ZnO and CaCO₃ as colorant provides an advantage vs. the composition of Comp. Ex. 2 comprising TiO₂ as white colorant.

### 1.9. Viscosity

Suspensions prepared with the formulations of Example 1, Comparative Example 1, and of Comparative Example 2 were studied in order to determine the range of viscosities of each formulation and its influence in the capsule manufacturing process.

As shown in Table 9, the viscosity was lower with the formulation of Example 1, which could facilitate the use of the suspension and the homogeneity within the dipping. pan.

**Table 9**

| | Comp. Ex. 1 | Comp. Ex. 2 (with TiO₂) | Ex. 1 (with ONFA) |
|---|---|---|---|
| Viscosity at 55°C (Pa.s) | 3.0 | 4.4 | 2.7 |

### 1.10. Rheology

The rheology and gelation of biodegradable polymers are of particular interest in the processing of films in pharmaceutical industry. Constant-stress temperature-ramps were used to determine the gelation temperature (Tgel).

To obtain this information, a Discovery Hybrid Rheometer-2 (DHR2) from TA Instruments, with a Peltier Concentric Cylinder system, and the TRIOS software, version 5.0.0.44616 was used. The method used was the following:

### Step 1) Flow-Peak Hold:

Temperature 55 °C Inherit Set Point: Off
Soak Time 180.0 s Wait For Temperature: On
Duration 30.0 s
Shear Rate 0.11/s
Inherit initial value: Off Sampling interval 1.0 s/pt
Stiff
Zero torque
Fast sampling: Off Save image: Off Enabled: Off Enabled: Off Enabled: Off

### Step 2) Flow-Peak Hold:

Temperature 55 °C Inherit Set Point: Off
Soak Time 0.0 s Wait For Temperature: On
Duration 30.0 s
Shear Rate 1.01/s
Inherit initial value: Off Sampling interval 1.0 s/pt
Stiff
Zero torque
Fast sampling: Off Save image: Off Enabled: Off Enabled: Off Enabled: Off

### Step 3) Flow-Peak Hold:

Temperature Inherit Set Point: On
Soak Time 0.0 s Wait For Temperature: Off
Duration 30.0 s
Shear Rate 10.01/s
Inherit initial value: Off Sampling interval 1.0 s/pt
Auto
Zero torque
Fast sampling: Off Save image: Off Enabled: Off Enabled: Off Enabled: Off

### Step 4) Flow-Peak Hold:

Temperature Inherit Set Point: On
Soak Time 0.0 s Wait For Temperature: Off
Duration 30.0 s

### Shear Rate 100.01/s

Inherit initial value: Off Sampling interval 1.0 s/pt
Stiff
Zero torque
Fast sampling: Off Save image: Off Enabled: Off Enabled: Off Enabled: Off

### Step 5) Flow-Peak Hold:

Temperature Inherit Set Point: On
Soak Time 0.0 s Wait For Temperature: Off
Duration 30.0 s
Shear Rate 10.01/s
Inherit initial value: Off Sampling interval 1.0 s/pt
Stiff
Zero torque
Fast sampling: Off Save image: Off Enabled: Off Enabled: Off Enabled: Off

### Step 6) Flow-Peak Hold:

Temperature Inherit Set Point: On
Soak Time 0.0 s Wait For Temperature: Off
Duration 30.0 s
Shear Rate 1.01/s
Inherit initial value: Off Sampling interval 1.0 s/pt
Stiff
Zero torque
Fast sampling: Off Save image: Off Enabled: Off Enabled: Off Enabled: Off

### Step 7) Flow-Peak Hold:

Temperature Inherit Set Point: On
Soak Time 0.0 s Wait For Temperature: Off
Duration 30.0 s
Shear Rate 0.11/s
Inherit initial value: Off Sampling interval 1.0 s/pt
Stiff
Zero torque
Fast sampling: Off Save image: Off Enabled: Off Enabled: Off Enabled: Off

### Step 8) Oscillation-Temperature Ramp:

Start temperature 55 °C Use entered value
Soak time 0.0 s Wait for temperature: Off
Ramp rate 1.0 °C/min
End temperature 15 °C
Soak time after ramp 0.0 s
Estimated time to complete 00:40:00 hh:mm:ss
Sampling interval 15.0 s/pt
Strain % 0.1%
Single point
Frequency 1.0 Hz
Non-iterative sampling
Torque 10.0 µN.m
Lower torque limit 10.0 µN.m
Number of tries 4
Initial tolerance 0.5 %
Store all tries as data points: Off
Store try information with point: Off
Time 3.0 s
Time 3.0 s
Save waveform (point display): On Number of points in waveform 64
Save image: Off Use additional harmonics: Off
Torque 0.0 µN.m
Enabled: Off Enabled: Off Enabled: Off

The gelling temperature of the suspension of the formulations of Example 1, Comparative Example 1, and of Comparative Example 2 mentioned above is shown in Table 10 below.

**Table 10**

| | Comp. Ex. 1 (uncolored) | Comp. Ex. 2 (with TiO₂) | Ex. 1 (with ONFA) |
|---|---|---|---|
| Tgel (°C) | 28.92 | 29.16 | 27.67 |

The gelling temperature of the suspension of the formulation of Example 1 is 27.67 °C. It is more that 1 °C lower than the ones of Comparative Example 1 and of Comparative Example 2. A lower gelling temperature can help to improve the process, allowing process temperatures to be lower, which would equate to shorter process times and lower energy consumption, as well as better control in drying. This also entails a reduced risk for the employee's safety.

In particular, the temperature of the suspension inside the dipping pan, as well as the resting temperature inside the tanks at the end of its preparation process, will be lower by at least 2 °C (from 55 °C with the suspension of the formulation of Comparative Example 2 to 53 °C with the suspension of the formulation of Example 1) and, consequently, the time to reach the gelling temperature will be also lower.

### 1.11. Process for the manufacture of the capsule shells

In a reactor provided with a planetary mixer and a cowles disperser, a first cellulose suspension of hypromellose, carrageenan (gelling agent), and KCI (gelling promoter) in water was prepared. Then, vacuum was applied to eliminate the possible bubbles generated in the process.

Additionally, two 30 wt.% aqueous suspensions of the white colorants, namely of TiO₂ and of ONFA, were also prepared with constant agitation.

Once the cellulose suspension was prepared, it was maintained at 55 °C and discharged in smaller tanks where it was mixed and blended with each one of the coloring suspension in order to obtain a suspension with the components of Comparative Example 2 and a suspension with the components of Example 1.

For the preparation of the suspension of the formulation of Comparative Example 2, the TiO₂ suspension was added to the cellulose suspension while maintaining the temperature of the suspension at 55 °C.

Similarly as above, for the preparation of the suspension of the formulation of Example 1, first, the cellulose suspension was cooled down to 53 °C and, then, the ONFA suspension was added to the cellulose suspension maintaining the mentioned temperature the rest of the process. Thus, the temperature for the rest of the process was 2 °C lower with the suspension of the formulation of Example 1 than with the suspension of the formulation of Comparative Example 2.

The tanks were connected to a capsule producing machine to enable the white colored cellulose suspension to reach two dipping pans, one for the cap of the capsule and another one for the body of the capsule.

The caps and the bodies of the capsule shells were produced by introducing a cap mold and a body mold in the corresponding dipping pan at 55 °C or 53 °C, depending on the formulation, and then spinning them to be in the opposite position to enter in the drying ovens. After being dried, the capsule caps and bodies were stripped off the mold and cut to the proper length. Then, the two halves (the cap and body) were joined to obtain the capsule shells.

## Claims

1. A TiO₂ free hard capsule shell comprising
(1) a water-soluble film forming compound selected from the group consisting of a water-soluble cellulose compound and gelatin,
(2) optionally, a gelling agent,
(3) optionally, a gelling aid,
(4) ZnO, and
(5) CaCO₃,
wherein ZnO and CaCO₃ are in a suitable amount to provide a white color to the film.

2. The hard capsule of claim 1, wherein CaCO₃ is precipitated calcium carbonate (PCC).

3. The hard capsule of claims 1 or 2, wherein the ZnO and CaCO₃ together are in an amount from 1 to 22 wt.%, preferably from 2 to 16 wt.%, more preferably from 3 to 14 wt.%, based on 100 wt.% of water-soluble film forming compound, optionally, gelling agent, optionally, the gelling aid, ZnO, and CaCO₃.

4. The hard capsule of claim 3, wherein ZnO is in an amount from 33 to 64 wt.% or from 50 to 76 wt.%, based on the total amount of ZnO and CaCO₃.

5. The hard capsule of any one of claims 1 to 4, wherein the water-soluble film forming compound is a water-soluble cellulose compound, and the hard capsule comprises a gelling agent.

6. The hard capsule of any one of claims 1 to 5, wherein the film forming compound is a water-soluble cellulose ether in which one or more hydrogen atoms of hydroxyl groups of cellulose are replaced with at least one group selected from the group consisting of alkyl and hydroxyalkyl, particularly hydroxypropyl methylcellulose.

7. The hard capsule of any one of claims 1 to 6, wherein the gelling agent is at least one member selected from the group consisting of carrageenan, pectin, xanthan gum, locust bean gum, tamarind seed polysaccharide, curdlan, gelatin, fur selenium, agar, and gellan gum.

8. The hard capsule of any one of claims 1 to 7, wherein the gelling aid is a compound capable of generating sodium ions, potassium ions, or calcium ions in an aqueous solution.

9. The hard capsule of claims 1 to 8, wherein the gelling agent is in an amount from 0.05 to 2 wt.% based on 100 wt.% of water-soluble film forming compound, gelling agent, optionally, the gelling aid, ZnO, and CaCO₃.

10. The hard capsule of claim 9, wherein the gelling aid is in an amount from 0.2 wt.% to 1 wt.% based on 100 wt.% of the total hard capsule components, excluding the aqueous solution.

11. The hard capsule of any one of claims 1 to 4, wherein the water-soluble film forming compound is gelatin.

12. The hard capsule of claims 1 to 11, wherein the film further comprises starch.

13. The hard capsule of claim 12, wherein starch is in an amount from 1 wt.% to 34 wt.%, based on 100 wt.% of the total amount of ZnO, CaCO₃ and starch.

14. A TiO₂ free hard capsule shell-preparing suspension comprising
(1) a water-soluble film forming compound selected from the group consisting of a water-soluble cellulose compound and gelatin,
(2) optionally, a gelling agent,
(3) optionally, a gelling aid,
(4) ZnO,
(5) CaCO₃, and
(6) an aqueous solvent,
wherein the suspension is for the preparation of a hard capsule shell, and ZnO and CaCO₃ are in a suitable amount to provide a white color to the film.

15. A capsule product comprising a TiO₂ free hard capsule as defined in any one of claims 1 to 13 filled with a formulation comprising an active ingredient.
